(19) Europäisches Patentamt / European Patent Office / Office européen des brevets

(11) **EP 4 312 722 B1**

(12) **EUROPEAN PATENT SPECIFICATION**

(45) Date of publication and mention of the grant of the patent:
**01.04.2026   Bulletin 2026/14**

(21) Application number: **22706287.4**

(22) Date of filing: **16.02.2022**

(51) International Patent Classification (IPC):
*A61B 5/00* *(2006.01)*      *A61B 5/021* *(2006.01)*
*A61B 5/024* *(2006.01)*

(52) Cooperative Patent Classification (CPC):
**A61B 5/7271; A61B 5/021; A61B 5/7232;**
**A61B 5/7235;** A61B 5/02416; A61B 5/346;
A61B 2560/0209

(86) International application number:
**PCT/EP2022/053762**

(87) International publication number:
**WO 2022/207177 (06.10.2022 Gazette 2022/40)**

(54) **MEDICAL DEVICE FOR DETERMINING AN EXTREMUM OF A PERIODIC PHYSIOLOGIC SIGNAL**

MEDIZINISCHE VORRICHTUNG ZUR BESTIMMUNG EINES EXTREMWERTS EINES PERIODISCHEN PHYSIOLOGISCHEN SIGNALS

DISPOSITIF MÉDICAL POUR DÉTERMINER UN EXTREMUM D'UN SIGNAL PHYSIOLOGIQUE PÉRIODIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **29.03.2021  EP 21165475**

(43) Date of publication of application:
**07.02.2024   Bulletin 2024/06**

(73) Proprietor: **BIOTRONIK SE & Co. KG**
**12359 Berlin (DE)**

(72) Inventors:
• **ELSNER, Joachim**
**14059 Berlin (DE)**
• **SKERL, Olaf**
**18209 Bad Doberan (DE)**
• **WEGERICH, Franziska**
**12055 Berlin (DE)**

(74) Representative: **Biotronik Corporate Services SE**
**Corporate Intellectual Property**
**Sieversufer 9**
**12359 Berlin (DE)**

(56) References cited:
EP-A1- 0 925 018      US-A1- 2011 201 946
US-A1- 2014 323 824

**Description**

The present invention relates to a medical device for determining an extremum of a periodic physiologic signal according to the preamble of claim 1, to a computer program product according to the preamble of claim 7, and to methods for determining an extremum of a periodic physiologic signal with a medical device according to the preamble of claims 8.

Conventional methods for signal evaluation are based on determining the periods of signal change and then determining the minima and maxima within these periods.

For the period determination, it is necessary to remove interferences and modulation influences from a determined signal. This requires extensive filtering of the signal and often extensive calculations of the signal components. For the determination of the periods of the cleaned signal, defined properties of the signal (e.g., zero crossings or inflection points) or characteristic structures in the signal (e.g., an R-wave of a cardiac signal) are determined with suitable means, up to known correlation methods.

There are also known methods by which one or more additional signals, often from additional sensors, are included to determine the signal periods (e.g., J. Kirchner, A. van Ooyen, S. Ershov and O. Skerl, "Enhancement of pulse contour analysis in the pulmonary artery by use of heart sounds," SENSORS, 2011 IEEE, Limerick, 2011, pp. 1792-1795).

After determining the signal periods, the minima and maxima are determined in each period and then averaged.

The known methods require computational effort and resources that cannot be provided in a miniaturized medical device and exceed the available energy budget of such miniaturized medical device.

According to another known method the data is not evaluated in the miniaturized medical device itself but outside in an additional device. For this purpose, the raw data is transmitted to the additional device without being processed. Large amounts of data generally have to be transmitted for this purpose. This data transmission also requires a high energy input.

The known methods require a high computing effort and extensive resources, which are difficult to implement in a miniaturized medical device. The resulting high energy expenditure often requires high capacities of the used energy storage, which often prevents sufficient miniaturization of the respective device or unreasonably limits the operating time of a miniaturized medical device.

This is illustrated by the following example: Assuming a pressure curve is to be transmitted from a sensor implant to an external device for evaluation purposes. In such a case, the pressure curve should be available in the external device as completely as possible so that the evaluation result is not distorted. This places high demands on transmission reliability (both for digital data transmission (as in case of active implants) and for analogue data transmission (as in case of passive implants)) and/or transmission accuracy (only for analogue data transmission (as in case of passive implants)).

A high energy budget is needed for a data transmission from an active implant. Such a high energy budget is not compatible with a battery capacity being typically less than 10 mAh and a lifetime of approximately 10 years, as can be seen from the following rough calculation.

Assuming that one pressure curve is transmitted every day over a total life time of 10 years. Further assuming that each pressure curve is recorded over a period of 10 seconds with 100 samples per second and a size of 12 bit per sample. Further considering that the limited maximum available battery capacity requires that a radio frequency transmitter is no longer active than 7.5 ms and the data transmission rate is limited to 16 kbit per second to guarantee sufficient data transmission reliability. This gives a maximum of 120 bit (15 bytes) per packet. Then, the individual data packets to be transferred need to be no longer than 5 byte (40 bit) because each data package typically requires 10 bytes (80 bit) for preamble, header and cyclic redundancy check (protocol overhead).

A maximum payload of 5 bytes results in 300 packets to be transferred. The transfer of each data packet (including protocol overhead) requires a time of 7.5 ms and a current of 5 mA. Thus, a capacity of 0.0375 mAs is needed for each data packet to be transferred, resulting in a required capacity of 11.25 mAs for data transfer of each pressure curve. In 10 years, 3650 pressure curves are recorded and transferred, resulting in a total needed capacity of 41062.5 mAs, i.e., 11.4 mAh. However, a typical miniaturized medical device only has a battery capacity of less than 10 mAh, wherein this battery capacity is not only needed for data transfer purposes, but also for other tasks to be accomplished by the miniaturized medical device.

Thus, miniaturized medical devices transferring raw data to an external device for evaluation purposes are not able to transfer one pressure curve per day over the whole lifetime considering the presently available state of miniaturization and the currently available battery capacities. In US 2011/0201946 A1, methods, systems and devices are provided for reducing the amount of data, processing and/or power required to analyze hemodynamic signals such as photoplethysmography (PPG) signals, pressure signals, and impedance signals. In response to detecting a specific event associated with a cyclical body function, a hemodynamic signal is continuously sampled during a window following the detecting of the specific event, wherein the window is shorter than a cycle associated with the cyclical body function. The hemodynamic signal is then analyzed based on the plurality of samples.

It is an object of the present invention to provide a medical device enabling a reliable data evaluation of measured physiologic data requiring less energy capacity than the devices known from prior art.

This object is achieved with a pressure sensor implant for determining an extremum of a periodic blood pressure signal having the features of claim 1. Such a pressure sensor implant comprises a computing unit, a memory unit, and a detecting unit configured to detect a periodic physiologic signal of a human or animal.

The memory unit comprises a computer-readable program that causes the computing unit to perform the steps explained in the following when executed on the computing unit.

In a first step, a periodic blood pressure signal is detected with a detecting unit. Many physiologic signals have a periodic variation in time, the parameters of which are modulated in dependence on surrounding conditions. In many cases, average values of minima and maxima of the individual signal periods within a given time interval are used as data for subsequent diagnostic purposes.

During or after detecting the periodic blood pressure signal, this periodic blood pressure signal is divided into a plurality of equally long intervals. Each of these intervals has an interval length defining the extension in time of each interval. The interval length is chosen such that it is longer than an expected maximum periodic time of the periodic blood pressure signal. To give an example, the heart rate of a human is typically higher than 60 beats per minute. At a heart rate of more than 60 beats per minute, the expected maximum periodic time is less than 1 second if the periodic blood pressure signal is the heartbeat or depends on the heartbeat. In such a case, the interval length is chosen to be equal to or longer than 1 second, e.g., 1.1 seconds, 1.2 seconds, 1.3 seconds, 1.4 seconds, 1.5 seconds, 1.6 seconds, 1.7 seconds, 1.8 seconds, 1.9 seconds, or 2.0 seconds. Expressed in other words, the interval length is chosen in this exemplary embodiment such that it lies between 1.0 second and 2.0 seconds. For this specific example, an interval length of 1.0, 1.25, or 1.5 seconds is particularly appropriate.

Due to the division of the periodic blood pressure signal into equally long (fixed) intervals, it is no longer necessary to determine individual signal periods. Such determination is comparatively complex and requires a lot of computing and energy capacity.

Afterwards, an absolute maximum and/or an absolute minimum of the periodic blood pressure signal are determined within each interval. Thus, a plurality of absolute maxima and/or a plurality of absolute minima results. The amount of absolute maxima or the amount of absolute minima corresponds to the number of intervals (i.e., there is exactly one absolute maximum and one absolute minimum in each interval).

Afterwards, an average value of the determined absolute maxima (if in the preceding step absolute maxima were determined) and/or an average value of the determined absolute minima (if in the preceding step absolute minima were determined) is calculated.

The calculated average value of the determined absolute maxima and/or the calculated average value of the determined absolute minima are stored or output as extremum or extrema of the periodic blood pressure signal. This extremum or these extrema represent original data that can be subsequently used by medical staff to make a diagnosis by comparing the obtained values with set (standard) values or evaluating derived trends.

By means of the presently described medical device, average values of extrema of a physiologic periodic signal (e.g., of the diastolic and systolic blood pressure) are available, wherein the required memory and computing capacity is only very low and furthermore only a minimum of energy is consumed for calculating the average values. Thus, diagnostic parameters or parameters representing original data for subsequent diagnosis can be directly determined within the (optionally miniaturized) medical device so that an energy-consuming data transfer of raw data to an external device is no longer necessary.

The method performed by the computer-readable program when executed on the computing unit can, in an embodiment, also be expressed in other words in the following way.

It is desired to find the average of the local minima of all periods of a periodic blood pressure signal within a first time period: $\overline{\mathrm{Min_{loc}}} = \frac{1}{N}\sum_{i=1}^{i=N}(\mathrm{Min_{loc}})_i$

The following procedure provides an approximate value Min.

  1. Subdivide a curve having a length T (in s) into N equal intervals $I_1 = [0 \dots T/N]$, $I_2 = [T/N \dots 2T/N]$, ..., $I_N = [(N-1)T/N \dots T]$, wherein $N \leq T^*f$. f (in 1/s) is the minimum average frequency that can typically be assumed for the periodic physiologic signal.

  2. Determine for each interval $I_i$, $1 \leq i \leq N$, the absolute minimum $(\mathrm{Min_{abs}})i$

  3. Calculate the mean value of the N absolute minima

  $\widetilde{\mathrm{Min}} = \frac{1}{N}\sum_{i=1}^{i=N}(\mathrm{Min_{abs}})_i i$  and use it as an approximation for $\overline{\mathrm{Min_{loc}}}$.

Similarly, absolute maxima can be used to get an approximation for the mean value of local maxima.

In an embodiment, the pressure sensor implant is a miniaturized implant having a battery with a battery capacity of less than battery capacity of less than 150 mAh, in particular of less than 100 mAh, in particular of less than 15 mAh, in particular of less than 10 mAh , in particular of less than 9 mAh, in particular of less than 8 mAh, in particular of less than 7 mAh, in particular of less than 6 mAh. In an embodiment, the battery capacity of the battery of the miniaturized implant lies within a range of 5 mAh to 10 mAh, in particular of 6 mAh to 9 mAh, in particular of 7 mAh to 8 mAh. Such miniaturized medical device typically has a lifetime of approximately 10 years. Thus, miniaturized implants are devices with limited resources that are characterized by an extremely small computing capacity and an extremely small energy consumption.

The presently claimed solution now provides a pressure sensor implant with novel enhanced data evaluation capabilities.

**In** an embodiment, the pressure sensor implant is a passive implant. Passive implants typically only sense or detect physiologic parameters of a human or animal and transfer them to an evaluation unit where they can be evaluated by medical staff. For this purpose, passive implants typically use analogue data transmission methods.

the periodic physiologic signal is a blood pressure signal.

Blood pressure, e.g., pulses with cardiac activity resulting in a diastolic blood pressure (minimum pressure within a heart cycle) and a systolic blood pressure (maximum pressure within a heart cycle). At the same time, the blood pressure is, if it is measured within the pulmonary artery, modulated by the respiration. This results in variations of the diastolic and systolic blood pressure. Therefore, diastolic and systolic pressure values are often averaged over a defined time to be then used as diagnostic parameters. By means of the presently described pressure sensor implant, such average values of the diastolic and systolic blood pressure are easily available also in miniaturized medical devices.

In an embodiment, the program causes the computing unit to perform the steps of detecting the periodic blood pressure signal, dividing the periodic blood pressure signal into a plurality of equally long intervals, determining at least one of an absolute maximum and an absolute minimum, calculating at least one average value, and storing or outputting the calculated average value without filtering a raw signal of the detected periodic blood pressure signal. Such filtering techniques often require a lot of computational resources and require a comparatively high amount of energy. These filtering techniques are no longer necessary if a device as presently described is used, making use of a simplified yet highly accurate determination of extrema in the periodic blood pressure signal.

In an embodiment, the program causes the computing unit to perform the steps of detecting the periodic blood pressure signal, dividing the periodic blood pressure signal into a plurality of equally long intervals, determining at least one of an absolute maximum and an absolute minimum, calculating at least one average value, and storing or outputting the calculated average value without smoothing the detected periodic blood pressure signal. While such smoothing is generally necessary according to data evaluation techniques known from prior art, it will not disturb the method implemented by the presently described and claimed pressure sensor implant. However, such smoothing is no longer necessary. The presently claimed and described device will also be able to evaluate the detected data without smoothing processes so that the computational effort, the necessary energy for evaluating the data and finally the time needed for providing the results of the data evaluation is significantly reduced with respect to prior art techniques.

The steps of dividing the detected periodic blood pressure signal and of determining the absolute maximum values and/or absolute minimum values does not depend on the curve morphology of the periodic physiologic signal. Thus, it is not necessary to perform "if/then/else" instructions during data analysis. Therefore, the computing unit can be, in an embodiment, an application-specific integrated circuit (ASIC), in particular a miniaturized ASIC (mini ASIC). In an alternative embodiment, the computing unit is a microprocessor, even though the computing capabilities of the microprocessor are not needed for the evaluation steps performed by the claimed pressure sensor implant.

In an aspect, the present invention relates to a computer program product comprising computer-readable code that causes a computing unit to perform the following steps when executed on the computing unit.

In a first step, a periodic blood pressure signal is detected with a detecting unit of a pressure sensor implant for determining an extremum of a periodic blood pressure signal.

During or after detecting the periodic blood pressure signal, this periodic blood pressure signal is divided into a plurality of equally long intervals. Each of these intervals has an interval length defining the extension in time of each interval. The interval length is chosen such that it is longer than an expected maximum periodic time of the periodic blood pressure signal.

Afterwards, an absolute maximum and/or an absolute minimum of the periodic blood pressure signal are determined within each interval. Thus, a plurality of absolute maxima and/or a plurality of absolute minima results. The amount of absolute maxima or the amount of absolute minima corresponds to the number of intervals (i.e., there is exactly one absolute maximum and one absolute minimum in each interval).

Afterwards, an average value of the determined absolute maxima (if in the preceding step absolute maxima were determined) and/or an average value of the determined absolute minima (if in the preceding step absolute minima were determined) is calculated.

The calculated average value of the determined absolute maxima and/or the calculated average value of the determined absolute minima are stored or output as extremum or extrema of the periodic blood pressure signal.

The present invention relates to a method for determining an extremum of a periodic blood pressure signal with pressure sensor implnat. Such a pressure sensor implant is typically implanted into the body of a human or animal. This method comprises the steps explained in the following.

In a first step, a periodic blood pressure signal is detected with a detecting unit of a pressure sensor implant for determining an extremum of a periodic blood pressure signal.

During or after detecting the periodic blood pressure signal, this periodic blood pressure signal is divided into a plurality of equally long intervals. Each of these intervals has an interval length defining the extension in time of each interval. The interval length is chosen such that it is longer than an expected maximum periodic time of the periodic blood pressure signal.

Afterwards, an absolute maximum and/or an absolute minimum of the periodic blood pressure signal are determined within each interval. Thus, a plurality of absolute maxima and/or a plurality of absolute minima results. The amount of absolute maxima or the amount of absolute minima corresponds to the number of intervals (i.e., there is exactly one absolute maximum and one absolute minimum in each interval).

Afterwards, an average value of the determined absolute maxima (if in the preceding step absolute maxima were determined) and/or an average value of the determined absolute minima (if in the preceding step absolute minima were determined) is calculated.

The calculated average value of the determined absolute maxima and/or the calculated average value of the determined absolute minima are stored or output as extremum or extrema of the periodic blood pressure signal. This extremum of these extrema represent original data that can be subsequently used by medical staff to make a diagnosis by comparing the obtained values with set (standard) values.

In a not claimed aspect, the present invention relates to a diagnostic method for making a diagnosis of a human or animal in need of such diagnosis, wherein the diagnostic method comprises the steps explained in the following.

First, an average value of absolute maxima and/or an average value of absolute minima of periodic physiologic signal is provided by carrying out a method according to the preceding explanations.

Afterwards, a deviation of the average value of absolute maxima and/or of the average value of absolute minima of the periodic physiologic signal from a given standard value is determined.

Based on this determined deviation, a diagnosis is made with respect to a physiologic parameter or the status of health of the human or animal.

All embodiments of the described medical device can be combined in any desired way and can be transferred either individually on any desired combination to the described computer program product and the described methods. Likewise, all embodiments of the described computer program product can be combined in any desired way and can be transferred either individually on any desired combination to the described medical device or the described methods. Finally, all embodiments of the described methods can be combined in any desired way and can be transferred either individually or in any arbitrary combination to the described medical device, to the respective other method, and to the described computer program product.

Further details of aspects of the present invention will be explained with respect to exemplary embodiments and accompanying Figures. In the Figures:

Fig. 1    shows the pressure developing in the pulmonary artery and in other body compartments;

Fig. 2    shows a first plot of a blood pressure curve;

Fig. 3    shows a second plot of the same blood pressure curve;

Fig. 4    shows a third plot of the same blood pressure curve; and

Fig. 5    shows a fourth plot of the same blood pressure curve.

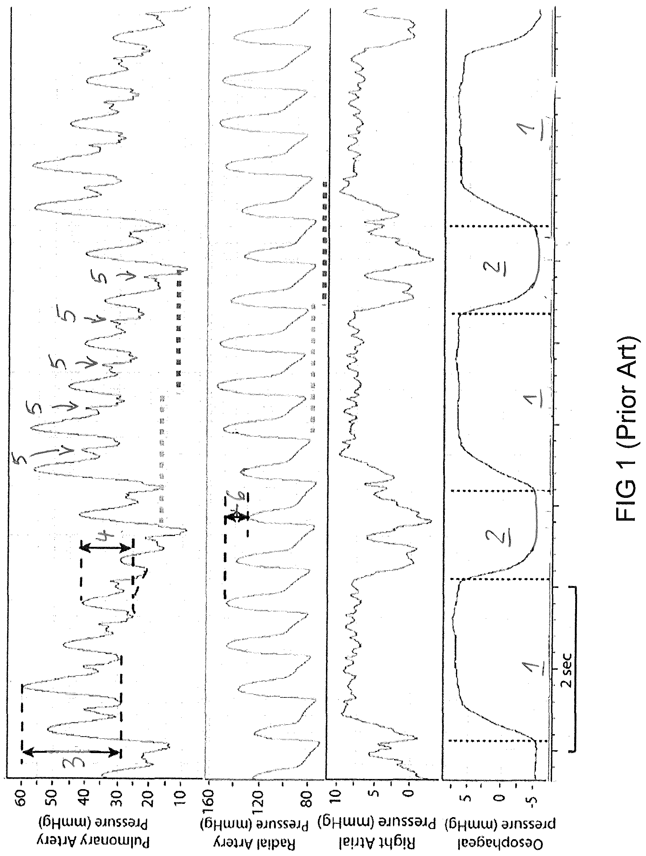

Figure 1 shows four plots of the developing of pressure values in different body compartments during a plurality of heart cycles. The topmost plot shows the pressure developing in the pulmonary artery, the second plot from the top the pressure developing in the radial artery, the third plot from the top the pressure developing in the right atrium, and the lowest plot the oesophageal pressure developing.

It is immediately apparent that the pulmonary artery pressure is modulated by expiration 1 and inspiration 2 (cf. topmost and lowest panel of Figure 1). During times of expiration 1, a first pulse 3 is much bigger than a second pulse 4 during inspiration 2. Consequently, the pulmonary artery pressure is bigger during times of expiration 1 than in times of inspiration 2. Therefore, it is not sufficient to determine an individual maximum or minimum of the pulmonary artery pressure to obtain the average systolic or diastolic pressure. Rather, an average needs to be calculated over a plurality of different pulmonary artery pressure signals. According to prior art techniques, this is done by determining all local maxima and local minima of individual periods of the pressure signal. This determination, however, is computing intensive, in particular if the pressure curve has a complex morphology. An applied algorithm can, e.g., wrongly interpret a valve closure 5 as systolic maximum or diastolic minimum. Please note in this circumstance that only some of the valve closures 5 are marked with the respective numeral reference.

Also when looking at the radial artery pressure in the second plot from the top, it is apparent that some pressure signals are much higher than other pressure signals. To give an example, a so-called pulsus paradoxus 6 (i.e., a pressure pulse being more than 10 mmHg smaller than the preceding pressure pulse) underlines the necessity not to rely on individual maxima or minima when determining the average systolic or diastolic pressure.

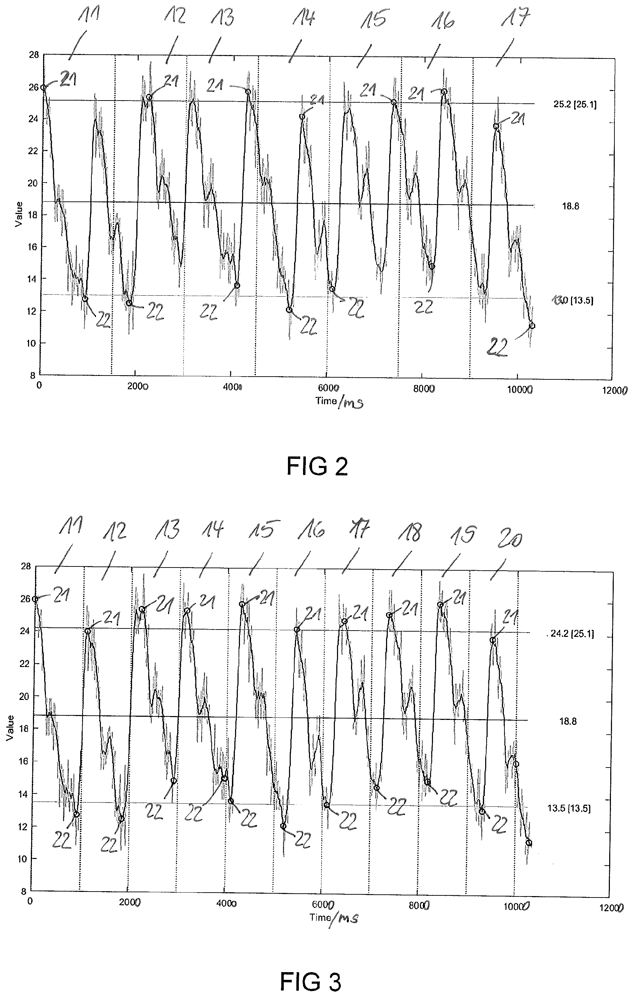

Figure 2 shows a typical blood pressure curve as exemplary embodiment of a periodic physiologic signal. This blood pressure extends curve over a time period of 10 seconds. It was obtained with a small pressure sensor implant implanted into the pulmonary artery of a human. This implant detects blood pressure signals with a sampling rate of 100 Hz.

In total, 1024 pressure values (10.24 s * 100/seconds) are chronologically stored in a memory of the pressure sensor implant. The expected minimum average heart rate $HR_{min}$ is defined to be more than 60 beats per minute, i.e., more than one beat per second. Consequently, N can be calculated as $N \leq T * HR_{min} = 10$ s * 1 s = 10.

This blood pressure curve is divided into seven equally long intervals 11, 12, 13, 14, 15, 16, 17, each of them lasting 1.5 seconds. In each of the intervals 11 to 17, an absolute maximum 21 and an absolute minimum 22 is determined. A pair of a maximum 21 and a minimum 22 located within a single of the intervals 11 to 17 does not necessarily belong to one and the same period of the blood pressure curve.

The black curve of Figure 2 represents the blood pressure signal being processed with a simple smoothing filter. This was done for better comparison with evaluation techniques known from prior art which require such a filtered signal. The blood pressure in Figures 2 to 5 as indicated in mmHg.

When applying the presently disclosed novel method for determining the average systolic blood pressure based on an average value of the determined maxima 21 and for determining the average diastolic blood pressure based on an average value of the determined minima 22, an average systolic blood pressure of 25.2 and an average diastolic blood pressure of 13.0 results. The mean average blood pressure is calculated to be 18.8. When applying a complex prior art method, an average systolic blood pressure of 25.1 and an average diastolic blood pressure of 13.5 is obtained (cf. values in brackets).

Thus, the average values obtained with the presently disclosed novel method are in good accordance with the values obtained with a much more complex method according to prior art.

Figure 3 shows the same blood pressure curve as in Figure 2. However, the intervals have been shortened from 1.5 seconds to only 1.0 seconds. Consequently, a total of 10 intervals 11, 12, 13, 14, 15, 16, 17, 18, 19, 20 results. The absolute maxima 21 and the absolute minima 22 are determined as explained with respect to Figure 2. Furthermore, in this and in all following Figures, the same numeral references will be used for similar elements.

By calculating average values from the absolute maxima 21 and the absolute minima 22, an average systolic blood pressure of 24.2 and an average diastolic blood pressure of 13.5 results. These values are still in good accordance with the values determined with a much more complex method according to prior art (cf. values in brackets).

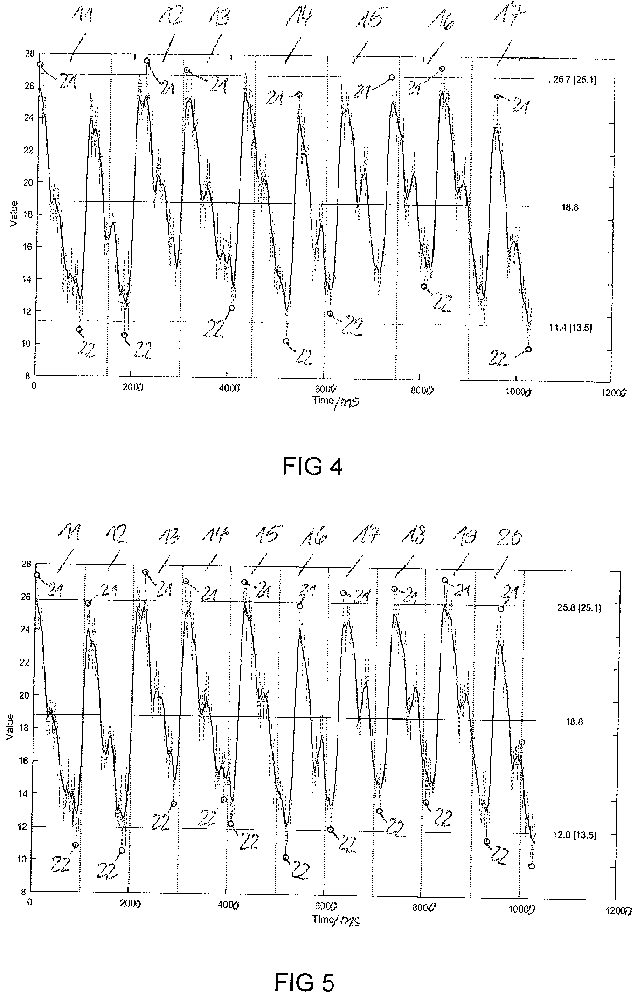

Figure 4 illustrates that the method described with respect to Figure 2 can also be applied to unfiltered raw signals. Once again, the blood pressure curve is divided into seven equally long intervals, each lasting 1.5 seconds. In contrast to Figure 2, the absolute maximum values 21 and the absolute minimum values 22 of each of the intervals 11 to 17 are not determined on the basis of a filtered or smoothed curve, but rather on the basis of the unfiltered and non-smoothed raw signal. An average systolic blood pressure of 26.7 and an average diastolic blood pressure of 11.4 results. The complex prior art technique does not allow use of unfiltered raw signals. The values for the average systolic blood pressure and the average diastolic blood pressure of 25.1 and 13.5 indicated in brackets in Figure 4 are only illustrated for comparative purposes. They have been calculated on the basis of a smoothened curve as explained with respect to and illustrated in Figure 2.

Figure 5 shows that also in case of using an unfiltered non-smoothed blood pressure curve, shortening of the interval length from 1.5 seconds to 1.0 seconds does not significantly distort the results of the average systolic blood pressure and the average diastolic blood pressure. Like in Figure 3, ten intervals 11 to 20 result. By calculating an average value for all maxima 21 of the individual intervals 11 to 20 results in an average systolic blood pressure of 25.8. Calculating an average of all minima 22 of the individual intervals 11 to 20 results in a diastolic blood pressure of 12.0.

Thus, the presently explained method does not only work with filtered and/or smoothed signals, but can rather also be applied to raw signals.

distort the results of the average systolic blood pressure and the average diastolic blood pressure. Like in Figure 3, ten intervals 11 to 20 result. By calculating an average value for all maxima 21 of the individual intervals 11 to 20 results in an average systolic blood pressure of 25.8. Calculating an average of all minima 22 of the individual intervals 11 to 20 results in a diastolic blood pressure of 12.0.

Thus, the presently explained method does not only work with filtered and/or smoothed signals, but can rather also be applied to raw signals.

**Claims**

1. Pressure sensor implant configured for implantation into the pulmonary artery for determining an extremum of a periodic blood pressure signal, comprising a computing unit, a memory unit, and a detecting unit configured to detect a blood pressure signal originating in the pulmonary artery of a human or animal, wherein the memory unit comprises a computer-readable program that causes the computing unit to perform the following steps when executed on the computing unit:

detecting the periodic blood pressure signal with the detecting unit,
dividing the periodic blood pressure signal into a plurality of equally long intervals (11, 12, 13, 14, 15, 16, 17, 18, 19, 20), each interval (11, 12, 13, 14, 15, 16, 17, 18, 19, 20) having an interval length, wherein the interval length is chosen such that it is longer than an expected maximum periodic time of the periodic blood pressure signal,
determining at least one of an absolute maximum (21) and an absolute minimum (22) of the periodic blood pressure signal within each interval (11, 12, 13, 14, 15, 16, 17, 18, 19, 20),
calculating at least one of an average value of the determined absolute maxima (21) and an average value of the determined absolute minima (22),
storing or outputting the calculated average value of the determined absolute maxima (21) and/or the calculated average value of the determined absolute minima (22) as extremum of the periodic blood pressure signal.

2. Pressure sensor implant according to claim 1, **characterized in that** the Pressure sensor implantis a miniaturized implant having a battery with a battery capacity of less than 150 mAh, in particular of less than 100 mAh, in particular of less than 15 mAh, in particular of less than 10 mAh.

3. Pressure sensor implantaccording to any of the preceding claims, **characterized in that** the implant is a passive implant.

4. Pressure sensor implantaccording to any of the preceding claims, **characterized in that** the program causes the computing unit to perform the steps of detecting the periodic blood pressure signal, dividing the periodic blood pressure signal into a plurality of equally long intervals (11, 12, 13, 14, 15, 16, 17, 18, 19, 20), determining at least one of an absolute maximum (21) and an absolute minimum (22), calculating at least one average value, and storing or outputting the calculated average value without filtering a raw signal of the detected periodic physiologic signal.

5. Pressure sensor implantaccording to any of the preceding claims, **characterized in that** the program causes the computing unit to perform the steps of detecting the periodic blood pressure signal, dividing the periodic blood pressure signal into a plurality of equally long intervals (11, 12, 13, 14, 15, 16, 17, 18, 19, 20), determining at least one of an absolute maximum (21) and an absolute minimum (22), calculating at least one average value, and storing or outputting the calculated average value without smoothing the detected periodic blood pressure signal.

6. Pressure sensor implantaccording to any of the preceding claims, **characterized in that** the computing unit is a microprocessor or an application-specific integrated circuit.

7. Computer program product comprising computer-readable code that causes a computing unit to perform the following steps when executed on the computing unit:

detecting a periodic blood pressure signal with a detecting unit of a pressure sensor implant for determining an extremum of a periodic blood pressure signal, dividing the periodic blood pressure signal into a plurality of equally long intervals (11, 12, 13, 14, 15, 16, 17, 18, 19, 20), each interval (11, 12, 13, 14, 15, 16, 17, 18, 19, 20) having an interval length, wherein the interval length is chosen such that it is longer than an expected maximum periodic time of the periodic blood pressure signal, determining at least one of an absolute maximum (21) and an absolute minimum (22) of the periodic blood pressure signal within each interval (11, 12, 13, 14, 15, 16, 17, 18, 19, 20),
calculating at least one of an average value of the determined absolute maxima (21) and an average value of the determined absolute minima (22),
storing or outputting the calculated average value of the determined absolute maxima (21) and/or the calculated average value of the determined absolute minima (22) as extremum of the periodic blood pressure signal.

8. Method for determining an extremum of a periodic blood pressure signal with an implanted pressure sensor implanted into the pulmonary artery of a human or an animal, the medical device comprising a computing unit, a memory unit, and a detecting unit configured to detect a periodic blood pressure signal of the human or animal, the method comprising the following steps:

detecting a periodic blood pressure signal with the detecting unit,
dividing the periodic blood pressure signal into a plurality of equally long intervals (11, 12, 13, 14, 15, 16, 17, 18, 19, 20), each interval (11, 12, 13, 14, 15, 16, 17, 18, 19, 20) having an interval length, wherein the interval length is chosen such that it is longer than an expected maximum periodic time of the periodic blood pressure signal,

determining at least one of an absolute maximum (21) and an absolute minimum (22) of the periodic blood pressure signal within each interval (11, 12, 13, 14, 15, 16, 17, 18, 19, 20),

calculating at least one of an average value of the determined absolute maxima (21) and an average value of the determined absolute minima (22),

storing or outputting the calculated average value of the determined absolute maxima (21) and/or the calculated average value of the determined absolute minima (22) as extremum of the periodic blood pressure signal.

**Patentansprüche**

1. Drucksensorimplantat, das zur Implantation in die Lungenarterie ausgestaltet ist, um ein Extremum eines periodischen Blutdrucksignals zu bestimmen, umfassend eine Recheneinheit, eine Speichereinheit und eine Erfassungseinheit, die zum Erfassen eines Blutdrucksignals aus der Lungenarterie eines Menschen oder Tieres ausgestaltet ist, wobei die Speichereinheit ein computerlesbares Programm umfasst, das die Recheneinheit veranlasst, die folgenden Schritte auszuführen, wenn es auf der Recheneinheit ausgeführt wird:

Erfassen des periodischen Blutdrucksignals mit der Erfassungseinheit,

Aufteilen des periodischen Blutdrucksignals in eine Vielzahl von gleich langen Intervallen (11, 12, 13, 14, 15, 16, 17, 18, 19, 20), wobei jedes Intervall (11, 12, 13, 14, 15, 16, 17, 18, 19, 20) eine Intervalllänge aufweist, wobei die Intervalllänge so gewählt ist, dass sie länger ist als eine erwartete maximale periodische Zeit des periodischen Blutdrucksignals,

Bestimmen wenigstens eines von einem absoluten Maximum (21) und einem absoluten Minimum (22) des periodischen Blutdrucksignals innerhalb jedes Intervalls (11, 12, 13, 14, 15, 16, 17, 18, 19, 20),

Berechnen wenigstens eines von einem Durchschnittswert der bestimmten absoluten Maxima (21) und einem Durchschnittswert der bestimmten absoluten Minima (22),

Speichern oder Ausgeben des berechneten Durchschnittswerts der bestimmten absoluten Maxima (21) und/oder des berechneten Durchschnittswerts der bestimmten absoluten Minima (22) als Extremum des periodischen Blutdrucksignals.

2. Drucksensorimplantat nach Anspruch 1, **dadurch gekennzeichnet, dass** das Drucksensorimplantat ein miniaturisiertes Implantat mit einer Batterie mit einer Batteriekapazität von weniger als 150 mAh, insbesondere von weniger als 100 mAh, insbesondere von weniger als 15 mAh, insbesondere von weniger als 10 mAh ist.

3. Drucksensorimplantat nach einem der vorangehenden Ansprüche, **dadurch gekennzeichnet, dass** das Implantat ein passives Implantat ist.

4. Drucksensorimplantat nach einem der vorangehenden Ansprüche, **dadurch gekennzeichnet, dass** das Programm die Recheneinheit veranlasst, die Schritte des Erfassens des periodischen Blutdrucksignals, des Aufteilens des periodischen Blutdrucksignals in eine Vielzahl von gleich langen Intervallen (11, 12, 13, 14, 15, 16, 17, 18, 19, 20), des Bestimmens wenigstens eines von einem absoluten Maximum (21) und einem absoluten Minimum (22), des Berechnens wenigstens eines Durchschnittswerts und des Speicherns oder Ausgebens des berechneten Durchschnittswerts ohne Filterung eines Rohsignals des erfassten periodischen physiologischen Signals auszuführen.

5. Drucksensorimplantat nach einem der vorangehenden Ansprüche, **dadurch gekennzeichnet, dass** das Programm die Recheneinheit veranlasst, die Schritte des Erfassens des periodischen Blutdrucksignals, des Aufteilens des periodischen Blutdrucksignals in eine Vielzahl von gleich langen Intervallen (11, 12, 13, 14, 15, 16, 17, 18, 19, 20), des Bestimmens wenigstens eines von einem absoluten Maximum (21) und einem absoluten Minimum (22), des Berechnens wenigstens eines Durchschnittswerts und des Speicherns oder Ausgebens des berechneten Durchschnittswerts ohne Glättung des erfassten periodischen Blutdrucksignals auszuführen.

6. Drucksensorimplantat nach einem der vorangehenden Ansprüche, **dadurch gekennzeichnet, dass** es sich bei der Recheneinheit um einen Mikroprozessor oder einen anwendungsspezifischen integrierten Schaltkreis handelt.

7. Computerprogrammprodukt, umfassend einen computerlesbaren Code, der eine Recheneinheit veranlasst, die folgenden Schritte auszuführen, wenn er auf der Recheneinheit ausgeführt wird:

Erfassen eines periodischen Blutdrucksignals mit einer Erfassungseinheit eines Drucksensorimplantats, um ein Extremum eines periodischen Blutdrucksignals zu bestimmen,

Aufteilen des periodischen Blutdrucksignals in eine Vielzahl von gleich langen Intervallen (11, 12, 13, 14, 15, 16, 17, 18, 19, 20), wobei jedes Intervall (11, 12, 13, 14, 15, 16, 17, 18, 19, 20) eine Intervalllänge aufweist, wobei die Intervalllänge so gewählt ist, dass sie länger ist als eine erwartete maximale periodische Zeit des periodischen Blutdrucksignals,

Bestimmen wenigstens eines von einem absoluten Maximum (21) und einem absoluten Minimum (22) des periodischen Blutdrucksignals innerhalb jedes Intervalls (11, 12, 13, 14, 15, 16, 17, 18, 19, 20),

Berechnen wenigstens eines von einem Durchschnittswert der bestimmten absoluten Maxima (21) und einem Durchschnittswert der bestimmten absoluten Minima (22),

Speichern oder Ausgeben des berechneten Durchschnittswerts der bestimmten absoluten Maxima (21) und/oder des berechneten Durchschnittswerts der bestimmten absoluten Minima (22) als Extremum des periodischen Blutdrucksignals.

**8.** Verfahren zum Bestimmen eines Extremums eines periodischen Blutdrucksignals mit einem in die Lungenarterie eines Menschen oder eines Tieres implantierten Drucksensor, wobei das medizinische Gerät eine Recheneinheit, eine Speichereinheit und eine Erfassungseinheit umfasst, die zum Erfassen eines periodischen Blutdrucksignals des Menschen oder Tieres ausgestaltet ist, wobei das Verfahren die folgenden Schritte umfasst:

Erfassen eines periodischen Blutdrucksignals mit der Erfassungseinheit,

Aufteilen des periodischen Blutdrucksignals in eine Vielzahl von gleich langen Intervallen (11, 12, 13, 14, 15, 16, 17, 18, 19, 20), wobei jedes Intervall (11, 12, 13, 14, 15, 16, 17, 18, 19, 20) eine Intervalllänge aufweist, wobei die Intervalllänge so gewählt ist, dass sie länger ist als eine erwartete maximale periodische Zeit des periodischen Blutdrucksignals,

Bestimmen wenigstens eines von einem absoluten Maximum (21) und einem absoluten Minimum (22) des periodischen Blutdrucksignals innerhalb jedes Intervalls (11, 12, 13, 14, 15, 16, 17, 18, 19, 20),

Berechnen wenigstens eines von einem Durchschnittswert der bestimmten absoluten Maxima (21) und einem Durchschnittswert der bestimmten absoluten Minima (22), Speichern oder Ausgeben des berechneten Durchschnittswerts der bestimmten absoluten Maxima (21) und/oder des berechneten Durchschnittswerts der bestimmten absoluten Minima (22) als Extremum des periodischen Blutdrucksignals.

## Revendications

**1.** Implant de capteur de pression configuré pour implantation dans l'artère pulmonaire afin de déterminer un extrémum d'un signal de pression artérielle périodique, comprenant une unité de calcul, une unité mémoire et une unité de détection configurée pour détecter un signal de pression artérielle ayant pour origine l'artère pulmonaire d'un humain ou d'un animal, dans lequel

l'unité de mémoire comprend un programme lisible par ordinateur qui amène l'unité de calcul à réaliser les étapes suivantes lorsqu'elles sont exécutées sur l'unité de calcul :

détecter le signal de pression artérielle périodique avec l'unité de détection, diviser le signal de pression artérielle périodique en une pluralité d'intervalles de longueur égale (11, 12, 13, 14, 15, 16, 17, 18, 19, 20), chaque intervalle (11, 12, 13, 14, 15, 16, 17, 18, 19, 20) ayant une longueur d'intervalle, dans lequel la longueur d'intervalle est choisie de sorte à être plus longue qu'une durée périodique maximale attendue du signal de pression artérielle périodique, déterminer au moins un parmi un maximum absolu (21) et un minimum absolu (22) du signal de pression artérielle périodique au sein de chaque intervalle (11, 12, 13, 14, 15, 16, 17, 18, 19, 20), calculer au moins une parmi une valeur moyenne des maximums absolus déterminés (21) et une valeur moyenne des minimums absolus déterminés (22), stocker ou émettre la valeur moyenne calculée des maximums absolus déterminés (21) et/ou la valeur moyenne calculée des minimums absolus déterminés (22) en tant qu'extrémum du signal de pression artérielle périodique.

**2.** Implant de capteur de pression selon la revendication 1, **caractérisé en ce que** l'implant de capteur de pression est un implant miniaturisé ayant une batterie avec une capacité de batterie de moins de 150 mAh, en particulier de moins de 100 mAh, en particulier de moins de 15 mAh, en particulier de moins de 10 mAh.

**3.** Implant de capteur de pression selon l'une quelconque des revendications précédentes, **caractérisé en ce que** l'implant est un implant passif.

**4.** Implant de capteur de pression selon l'une quelconque des revendications précédentes, **caractérisé en ce que** le programme amène l'unité de calcul à réaliser les étapes consistant à détecter le signal de pression artérielle périodique, diviser le signal de pression artérielle périodique en une pluralité d'intervalles de longueur égale (11, 12, 13, 14, 15, 16, 17, 18, 19, 20), déterminer au moins un parmi un maximum absolu (21) et un minimum absolu (22), calculer au moins une valeur moyenne, et stocker ou émettre la valeur moyenne calculée sans filtrer un signal brut du signal physiologique périodique détecté.

5. Implant de capteur de pression selon l'une quelconque des revendications précédentes, **caractérisé en ce que** le programme amène l'unité de calcul à réaliser les étapes consistant à détecter le signal de pression artérielle périodique, diviser le signal de pression artérielle périodique en une pluralité d'intervalles de longueur égale (11, 12, 13, 14, 15, 16, 17, 18, 19, 20), déterminer au moins un parmi un maximum absolu (21) et un minimum absolu (22), calculer au moins une valeur moyenne, et stocker ou émettre la valeur moyenne calculée sans lisser le signal de pression artérielle périodique détecté.

6. Implant de capteur de pression selon l'une quelconque des revendications précédentes, **caractérisé en ce que** l'unité de calcul est un microprocesseur ou un circuit intégré spécifique de l'application.

7. Produit de programme informatique comprenant un code lisible par ordinateur qui amène une unité de calcul à réaliser les étapes suivantes lorsqu'elles sont exécutées sur l'unité de calcul :

    détecter un signal de pression artérielle périodique avec une unité de détection d'un implant de capteur de pression pour déterminer un extrémum d'un signal de pression artérielle périodique,
    diviser le signal de pression artérielle périodique en une pluralité d'intervalles de longueur égale (11, 12, 13, 14, 15, 16, 17, 18, 19, 20), chaque intervalle (11, 12, 13, 14, 15, 16, 17, 18, 19, 20) ayant une longueur d'intervalle, dans lequel la longueur d'intervalle est choisie de sorte à être plus longue qu'une durée périodique maximale attendue du signal de pression artérielle périodique,
    déterminer au moins un parmi un maximum absolu (21) et un minimum absolu (22) du signal de pression artérielle périodique au sein de chaque intervalle (11, 12, 13, 14, 15, 16, 17, 18, 19, 20),
    calculer au moins une parmi une valeur moyenne des maximums absolus déterminés (21) et une valeur moyenne des minimums absolus déterminés (22),
    stocker ou émettre la valeur moyenne calculée des maximums absolus déterminés (21) et/ou la valeur moyenne calculée des minimums absolus déterminés (22) en tant qu'extrémum du signal de pression artérielle périodique.

8. Procédé de détermination d'un extrémum d'un signal de pression artérielle périodique avec un capteur de pression implanté dans l'artère pulmonaire d'un humain ou d'un animal,
le dispositif médical comprenant une unité de calcul, une unité mémoire et une unité de détection configurée pour détecter un signal de pression artérielle périodique de l'humain ou de l'animal, le procédé comprenant les étapes suivantes :

    détecter un signal de pression artérielle périodique avec l'unité de détection, diviser le signal de pression artérielle périodique en une pluralité d'intervalles de longueur égale (11, 12, 13, 14, 15, 16, 17, 18, 19, 20), chaque intervalle (11, 12, 13, 14, 15, 16, 17, 18, 19, 20) ayant une longueur d'intervalle, dans lequel la longueur d'intervalle est choisie de sorte à être plus longue qu'une durée périodique maximale attendue du signal de pression artérielle périodique, déterminer au moins un parmi un maximum absolu (21) et un minimum absolu (22) du signal de pression artérielle périodique au sein de chaque intervalle (11, 12, 13, 14, 15, 16, 17, 18, 19, 20),
    calculer au moins une parmi une valeur moyenne des maximums absolus déterminés (21) et une valeur moyenne des minimums absolus déterminés (22), stocker ou émettre la valeur moyenne calculée des maximums absolus déterminés (21) et/ou la valeur moyenne calculée des minimums absolus déterminés (22) en tant qu'extrémum du signal de pression artérielle périodique.

FIG 1 (Prior Art)

FIG 2

FIG 3

FIG 4

FIG 5

13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110201946 A1

**Non-patent literature cited in the description**

- Enhancement of pulse contour analysis in the pulmonary artery by use of heart sounds. **J. KIRCHNER** ; **A. VAN OOYEN** ; **S. ERSHOV** ; **O. SKERL**. SENSORS. IEEE, 2011, 1792-1795